# EUROPEAN PATENT SPECIFICATION

(11) **EP 4 519 653 B1**
(45) Date of publication and mention of the grant of the patent: **28.10.2026**
(21) Application number: 22736353.8
(22) Date of filing: 06.05.2022
(51) Int. Cl.: G01N 15/08, G01N 33/24

(54) **ROCK PROPERTIES MEASUREMENT CELL AND ASSOCIATED ROCK PROPERTIES MEASUREMENT SYSTEM**
MESSZELLE FÜR GESTEINSEIGENSCHAFTEN UND ZUGEHÖRIGES MESSSYSTEM FÜR GESTEINSEIGENSCHAFTEN
CELLULE DE MESURE DES PROPRIÉTÉS DES ROCHES ET SYSTÈME DE MESURE DES PROPRIÉTÉS DES ROCHES ASSOCIÉ

(43) Date of publication of application: 12.03.2025
(73) Proprietor: TotalEnergies OneTech, 92400 Courbevoie (FR)
(72) Inventor: DE OLIVEIRA FERNANDES, Victor, 64018 Pau Cedex (FR); CAUBIT, Cyril, 64018 Pau Cedex (FR); BRUGIDOU, Régis, 64400 Gurmencon (FR); NONO NGUENDJIO, Frank Laurel, 64018 Pau Cedex (FR)
(74) Representative: Lavoix
(86) International application number: PCT/IB2022/000270
(87) International publication number: WO 2023/214193

(56) References cited:
- US-A- 5 297 420
- US-A1- 2018 335 374
- US-B2- 8 024 960

## Description

The invention relates to a rock properties measurement cell.

In the domain of oil and gas exploration, it is critical to define the properties of rocks that are located at an exploration site, since local properties to the rock impact the overall behavior of the explored site. Such properties can include, for example, relative permeability or wettability of the rock.

In order to define properties of the rock, a rock sample is in general extracted from the exploration site and several tests are conducted on the rock sample mounted in a rock properties measurement cell, in order to define the rock properties.

US 5 297 420 A discloses an example of a rock property testing apparatus.

To properly characterize the properties of the rock, and incidentally, the behavior of the exploration site, it is generally required to prepare the rock sample so that it exhibits a target initial first fluid saturation as it is the case at its location in the reservoir, with a profile along a rock sample axis. Such initial first fluid saturation profile is generally an initial water saturation profile, often referred to as "Swi", and correspond to a proportion of water relatively to the overall fluid present in the rock (for example mix of the first fluid with a second fluid such as oil), along the rock sample axis.

A first type of known measurement cell to measure the target first fluid saturation profile comprise a body defining a chamber where the rock sample is located. The rock sample is flooded with fluid from an inlet face of the rock sample and fluid is directly retrieved from an outlet face of the rock sample. Such measurement cells allows quickly obtaining a first fluid saturation profile along the rock sample axis but the obtained first fluid saturation profile is not homogenous.

A second type of measurement cells generally comprise a monolithic porous barrier element that define a measurement chamber where rock sample is received. The monolithic porous barrier element is generally a disk located downstream the rock sample, that is permeable to a first fluid and impervious to a second fluid. Using such a porous barrier element allows obtaining a first fluid saturation profile along the rock sample axis that is homogenous; however, this operation is extremely time demanding. A goal of the invention is thus to provide a rock properties measurement cell that allows switching between different flooding schemes of the rock sample axis.

To that end, the invention relates to a rock properties measurement cell according to claim 1.

Such a rock properties measurement cell allows flexibility between retrieving first and/or second fluids directly from the rock sample through the bypass or retrieving only first fluid through the porous barrier element, thus allowing the implementation of different flooding schemes.

According to specific embodiments of the invention, the rock properties measurement cell further presents one or several of the features corresponding to claims 2 to 9, considered independently or along any technically possible combination.

The invention relates to a rock properties measurement system according to claim 10.

According to specific embodiments of the invention, the rock properties measurement system further presents one or several of the features corresponding to claims 11 to 13, considered independently or along any technically possible combination.

Other features and advantages of the invention will become apparent from a detailed description which is given thereof below, as an indication and by no means as a limitation, with reference to the appended figures, wherein:
- Figure 1 is a schematic representation of a rock properties measurement system according to the invention;
- Figure 2 is a schematic representation of the rock properties measurement cell of the rock properties measurement system of figure 1;
- Figure 3 is a graph of an evolution of the first fluid saturation profile during a primary flooding implemented in the rock properties measurement cell of figure 2;
- Figure 4 is a graph of an evolution of the first fluid saturation profile during a secondary flooding implemented in the rock properties measurement cell of figure 2; and

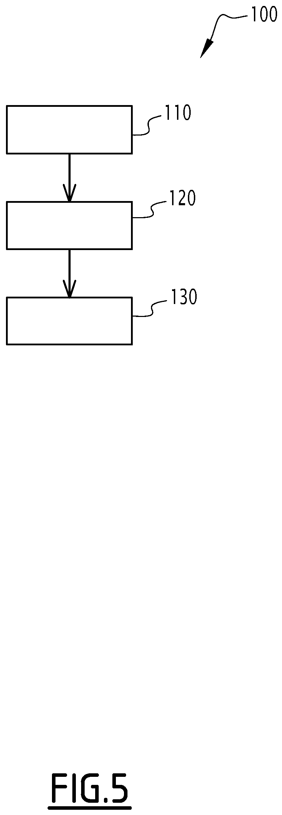

- Figure 5 is an organigram of the steps of a method for preparing a rock sample with a target initial first fluid saturation profile along a rock sample axis, implemented by the rock properties measurement system of figure 1.

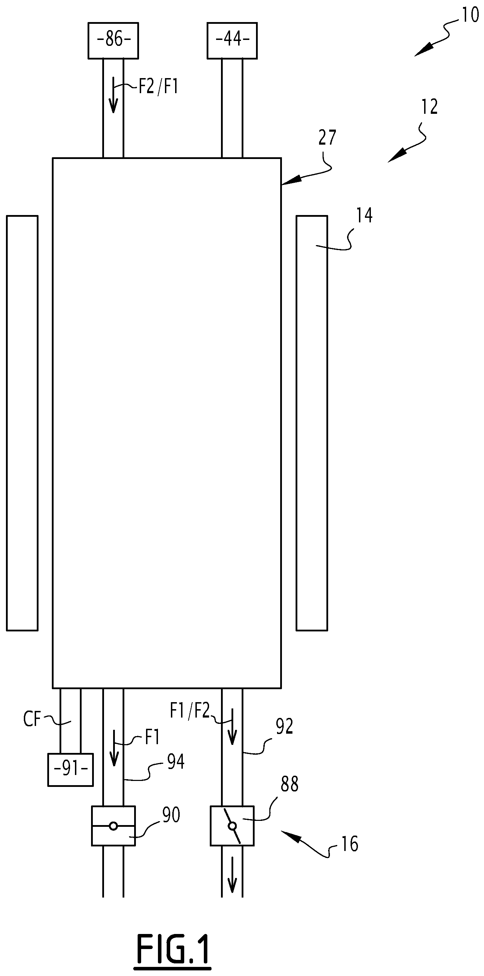

Referring to figure 1, a rock properties measurement system 10 comprises a rock properties measurement cell 12, an inner structure imaging device 14 and a fluid regulation arrangement 16.

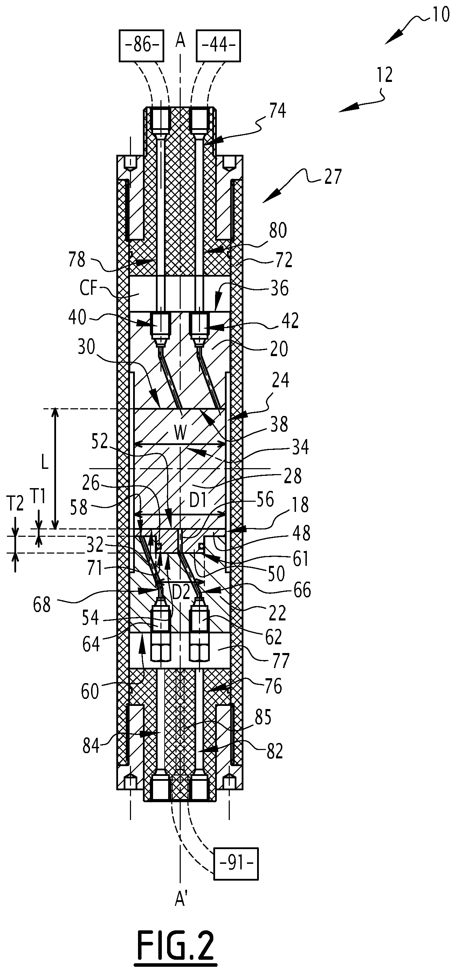

An example of the rock properties measurement cell 12 is illustrated on figure 2. The rock properties measurement cell 12 comprises a monolithic porous barrier element 18, an upstream diffuser 20, a downstream diffuser 22 and a sleeve 24.

The rock properties measurement cell 12 further preferably comprise a sealing gasket 26 and a confinement housing 27.

The rock properties measurement cell 12 is configured for receiving a rock sample 28 to carry out inner structure imaging of at least a fluid F1, F2, contained in said rock sample 28.

The rock properties measurement cell 12 is in particular configured for receiving a rock sample 28 to carry out measurement of a saturation profile S1i of a first fluid in in the rock sample.

In the rest of the description, the terms upstream and downstream are understood by reference with the displacement of the at least a fluid F1, F2, within the rock properties measurement cell 12, that is an upstream element being located higher on the figure 2 than a downstream element.

As this will be exposed later, the first fluid F1 is preferably water and/or the second fluid is preferably oil.

In particular, the first fluid is preferably water including at least one salt such as a brine with a salt concentration comprised between 1 g/l and 300 g/l. The water including at least one salt may comprise a plurality of salts of different compositions.

The second fluid is for example an oil or a gas chosen from the following types of oils and gas : mineral synthetic oil, dead oil, live oil, Nitrogen, compressed air, Helium.

In alternative, and for example, the first fluid F1 is oil and/or the second fluid F2 is water.

It will be understood that first F1 and/or second F2 fluids can also be other fluids differing from water and oil.

As seen on figure 2, the upstream diffuser 20, the rock sample 28, the porous element 18 and the downstream diffuser 22 are aligned in this order in a flow direction of the at least one fluid F1, F2.

The rock sample 28 is of any type and is for example a rock sample that is extracted from an oil and gas exploration site. In some specific examples, the permeability of the rock sample is low, that is the permeability of the rock sample is for example under 1 mD (milliDarcy).

The rock sample 28 defines a rock sample axis A-A' and is preferably elongated along the rock sample axis A-A'.

The rock sample 28 comprises an inlet face 30 and an outlet face 32. The inlet face 30 and the outlet 32 face are opposed relatively to the rock sample axis A-A' and are transverse to the rock sample axis A-A'.

In the example of figure 2, the rock sample 28 is cylindrical.

A length L of the rock sample 28 along the rock sample axis A-A' is for example comprised between 20 mm and 300 mm.

The width W of the rock sample 28, taken transversally to the rock sample axis A-A', is for example comprised between 10mm and 75 mm.

As illustrated in figure 2, the rock sample 28 is received in measurement chamber 34 of the measurement cell 12.

The measurement chamber 34 is delimited by the upstream diffuser 20, the porous barrier element 18 and the sleeve 24.

The dimension of the measurement chamber 34 is for example similar to the dimensions of the rock sample 28. The rock sample 28 is thus for example in contact with the upstream diffuser 20, the porous barrier element 18 and the sleeve 24 when the rock sample 28 is received in the measurement chamber 34.

The sleeve 24 is arranged around the upstream diffuser 20, the downstream diffuser 22 and the porous barrier element 18. As illustrated in figure 1, the sleeve 24 is arranged around the porous barrier element 18 to surround the entire porous barrier element 18 along the rock sample axis A-A' and is arranged to at least partially surround the upstream diffuser 20 and the downstream diffuser 22.

The sleeve 24 is preferably arranged to surround the rock sample 28 along the rock sample axis A-A'. In the example of figure 2 where the rock sample 28 is cylindrical, the sleeve 24 is a tubular sleeve 24.

The inner diameter of the sleeve 24 therefore sensibly corresponds to the width W of the rock sample 28, the inner diameter of the sleeve 24 being inferior or equal to the width W of the rock sample 28.

For example, the inner diameter of the sleeve 24 is equal to 8 mm when the width W of the rock sample 28 is equal to 10 mm, and is equal to 36 mm when the width W of the rock sample 28 is equal to 38 mm, the elasticity of the sleeve 24 allowing the sleeve to be arranged around the rock sample 28.

The sleeve 24 is arranged to surround the barrier element 18.

As seen on figure 2 the sleeve 24 preferably protrudes on both sides of the rock sample 28 along the rock sample axis A-A' so that is also surround at least a partially the upstream diffuser 20 and the downstream diffuser 22.

A thickness of the sleeve 24 is comprised between 0.5mm and 5mm and is for example equal to 1 mm.

The sleeve 24 is transparent to inner structure imaging such as microtomography imaging and/or nuclear magnetic resonance imaging.

The sleeve 24 is impervious to the fluids present in the rock sample F1, F2 so that the sleeve 24 guides the fluids F1 and/or F2 along the rock sample axis A-A' within the rock sample 28.

The sleeve 24 is for example made of fluorocarbon-based fluoroelastomer. The sleeve 24 is for example made of Viton^{®}, a fluorocarbon-based fluoroelastomer developed by the company DuPont.

As it will be presented later, the sleeve 24 is for example pressed around the upstream diffuser 20, the downstream diffuser 22, the porous barrier element 18 and the rock sample 28 received in the measurement chamber 34 when the measurement cell 12 is used to measure rock properties.

The upstream diffuser 20 is aligned to the rock sample axis and is located upstream of the measurement chamber 34, or in other terms, upstream of the rock sample 28.

The upstream diffuser 20 is preferably transparent to inner structure imaging such as microtomography imaging and/or nuclear magnetic resonance imaging.

The upstream diffuser 20 is for example made of a poly ketone polymer, in particular polyether ether ketone.

The upstream diffuser 20 is configured to guide at least second fluid to the rock sample 28.

The upstream diffuser 20 comprises an upstream side 36 and a downstream side 38 opposed to the upstream side 36 relatively to the upstream diffuser 20.

The upstream diffuser 20 also comprises a first upstream opening 40 and comprises for example a second upstream opening 42.

The first upstream opening 40 extends through the upstream diffuser 20 to connect the downstream side 38 and the upstream side 36 of the upstream diffuser 20.

The first upstream opening 40 is configured for guiding the second fluid F2 into the rock sample from the upstream side 36 to the downstream side 38 of the upstream diffuser 20.

In the example of figure 2, the first upstream opening 40 is angled relatively to the rock sample axis A-A'. The angle defined between the first upstream opening 40 and the rock sample axis is for example comprised between 0° and 45°, for example between 5° and 30° .

The second upstream opening 42 extends through the upstream diffuser 20 to connect the downstream side 38 and the upstream side 36 of the upstream diffuser 20.

The second upstream opening 42 is configured for fluidically connecting the rock sample with a pressure measurement device 44 of the fluid regulation arrangement 16.

In the example of figure 2, the second upstream opening 42 is angled relatively to the rock sample axis A-A' similarly to the first upstream opening 42.

In a particular example, the downstream side 38 of the upstream diffuser 20 comprises at least one groove (not shown) connected to the first upstream opening 40. The groove extends for example around the rock sample axis A-A' and is configured to guide the second fluid F2 along the inlet face 30 of the rock sample 28.

As shown on figure 2, a downstream portion of the diffuser has a smaller radial section than an upstream portion of the upstream diffuser 20. The downstream portion of the upstream diffuser 20 is for example surrounded by the sleeve 24.

The monolithic porous barrier element 18 is aligned to the rock sample axis A-A' and is located downstream of the measurement chamber 34, or in other terms, downstream of the rock sample 28.

The monolithic porous barrier element 18 is for example made of ceramic and for example an alpha alumina ceramic.

Pores size of the monolithic porous barrier element 18 is for example comprised between 20nm and 20nm and is for example equal to 50nm, 80nm or 150nm.

The monolithic porous barrier element is for example formed from an oil wet ceramic or a water wet ceramic.

The monolithic porous barrier element 18 is permeable to the first fluid F1 and impervious to the second fluid F2. In other words, when a first fluid F1 and a second fluid F2 are located within the rock sample 28, only the first fluid F1 can be retrieved from the rock sample 28 through the porous barrier element 18.

The monolithic porous barrier element 18 comprises an upstream segment 48 of a first diameter D1 and a downstream segment 50 of a second diameter D2. The second diameter D2 is smaller than the first diameter D1. In a median cross section, the upstream segment 48 and the downstream segment 50 define a T shape of the monolithic porous barrier element 18.

The upstream segment 48 defines an upstream face 52 of the rock sample. As visible on figure 2, the upstream face 52 is configured to cooperate with the rock sample 28, in particular such that the outlet face 32 of the rock sample 28 is in contact with the upstream face 52 of the porous barrier element 18.

As visible on figure 2, the upstream face 52 is for example essentially flat. Alternatively, and for example, the upstream face 52 is substantially flat and comprises at least one groove (not illustrated).

The upstream 48 and downstream 50 segments define together a downstream face 54 opposed to the upstream face 52. As shown on figure 2, the downstream face 54 therefore comprise two annular sub-faces connected together by a cylindrical sub-face.

The porous barrier element 18 comprises a bypass 56 connecting the upstream face 52 and the downstream face 54 through both the upstream segment 48 and the downstream segment 50. Thus, when a first fluid F1 and a second fluid F2 are located within the rock sample 28, both the first fluid F1 and the second fluid F2 can be retrieved from the rock sample 28 through the bypass 56.

In the example presented in figure 2, the porous barrier element 18 is a revolution element and the bypass 56 extends through the porous barrier element 18 following the axis of revolution of the element. In particular, the bypass 56 is aligned to the rock sample axis A-A'.

The first diameter D1 preferably corresponds to the width W of the rock sample axis. The thickness T1 of the upstream segment is preferably comprised between 3mm and 20mm.

The ratio of the thickness T1 over the diameter D1 of the upstream segment 48 is for example smaller than 40%.

The thickness T2 of the downstream segment is preferably comprised between 5mm and 50mm.

The downstream diffuser 22 is aligned to the rock sample axis A-A' and is located downstream of the porous element barrier element 18.

The downstream diffuser 22 is preferably transparent to inner structure imaging such as microtomography imaging and/or nuclear magnetic resonance imaging.

The downstream diffuser 22 is for example made of a poly ketone polymer, in particular polyether ether ketone.

The downstream diffuser 22 comprises an upstream side 58 and a downstream side 60 opposed to the upstream side 56 relatively to the downstream diffuser 22.

The downstream side 60 of the downstream diffuser 22 is for example essentially flat. Alternatively, and for example, the downstream side 60 of the downstream diffuser 22 is substantially flat and comprises at least one groove (not illustrated).

As illustrated in figure 2, the downstream diffuser 22 defines a cavity 61.

The cavity 61 is delimited by the upstream side 58 of the downstream diffuser 22. As shown on figure 2, the upstream side 58 therefore comprise two annular sub-faces connected together by a cylindrical sub-face.

The cavity 61 is conformed to receive the porous barrier element 18. In particular, and as shown on figure 2, the downstream segment 50 of the porous element 18 is received in the cavity 61, the shape of the cavity 61 being complementary to the shape of the downstream segment 50 of the porous element 18.

The downstream diffuser 22 comprises a first downstream opening 62 and a second downstream opening 64.

The first downstream opening 62 is for example aligned with the bypass, that is, the first downstream opening 62 is connected to the bypass 56. The first downstream opening 62 delimits together with the bypass 56 a first open channel 66.

In the example of figure 2, the first downstream opening 62 is angled relatively to the rock sample axis A-A'. The angle defined between the first downstream opening 62 and the rock sample axis is for example comprised between 0° and 45°, for example between 5° and 30°.

The first open channel 66 opens directly on the rock sample 28 and extends through the downstream diffuser 22 and the barrier element 18.

The first open channel 66 is configured to retrieve first F1 and/or second F2 fluid from the rock sample 28.

The second downstream opening 64 forms a second open channel 68.

In the example of figure 2, the second downstream opening 64 is angled relatively to the rock sample axis A-A' similarly to the first downstream opening 62.

The second open channel 68, opens facing the porous barrier element 18 and in particular facing the upstream segment 48 on the downstream face 54.

The second open channel 68 in configured to retrieve first fluid from the rock sample 28. In particular, the second open channel 68 is configured to retrieve only the first fluid F1 from the rock sample 28 that passed through the porous barrier element 18.

As presented in figure 2, the sealing gasket 26 is arranged around the downstream segment 50 of the porous element 18.

The sealing gasket 26 is arranged between, preferably radially between, the downstream segment 50 and the downstream diffuser 22.

In a preferred embodiment, the porous element comprises an annular peripheral housing 71 the sealing gasket 26 being arranged in the annular peripheral housing. In a non-illustrated embodiment, the sealing gasket 26 is instead arranged in an annular peripheral housing of the downstream diffuser 22.

As illustrated in figure 2, the confinement housing 27 is arranged around the sleeve 24. Since the sleeve 24 is arranged at least partially around the upstream diffuser 20, the downstream diffuser 22 and the porous barrier element 18, the confinement housing 27 is also arranged around the upstream diffuser 20, the downstream diffuser 22 and the porous barrier element 18, and around the sample 28 received in the measurement chamber 34.

The confinement housing 27 is transparent to inner structure imaging such as microtomography imaging and/or nuclear magnetic resonance imaging.

The confinement housing 27 is for example made of a poly ketone polymer, in particular polyether ether ketone.

The confinement housing 27 comprises a confinement frame 72, a upstream cap 74 and a downstream cap 76.

The confinement housing 27 is configured to receive a confinement fluid CF to apply pressure to the sleeve 24 around the upstream diffuser 20, the downstream diffuser 22, the porous barrier element 18 and the rock sample 28 received in the measurement chamber 34.

As seen in figure 2, the confinement fluid CF is contained by the confinement housing 27 in a confinement space 77 arranged between the confinement housing 27 on one side and the sleeve 24, the upstream diffuser 20 and the downstream diffuser 22 on the other side.

The confinement fluid CF is transparent to inner structure imaging such as microtomography imaging and/or nuclear magnetic resonance imaging.

The confinement fluid CF is for example water, mineral oil, nitrogen and for example a fluorinated oil.

The confinement frame 72 is for example tubular, the confinement frame being aligned with the rock sample axis A-A'.

The upstream cap 74 comprises a first upstream tubing 78 and preferably comprises a second upstream tubing 80. As shown in figure 2, the upstream cap 74 is for example made of two distinct parts.

The first upstream tubing 78 is fluidically connected to the first upstream opening 40. In particular, the first upstream tubing 78 fluidically connects the first upstream opening 40 to an outside of the measurement cell 12 and in particular fluidically connects the first upstream opening 40 with the fluid regulation arrangement 16 to guide first fluid F1 and/or second fluid F2 into the rock sample 28.

The second upstream tubing 80 is fluidically connected to the second upstream opening 42. In particular, the second upstream tubing 80 fluidically connects the second upstream opening 42 to the outside of the measurement cell 12 and in particular fluidically connects the second upstream opening 42 with the pressure measurement device 44 of the fluid regulation arrangement 16.

The downstream cap 76 comprises a first downstream tubing 82 and a second downstream tubing 84. As shown in figure 2, the downstream cap 76 is for example made of two distinct parts.

The first downstream tubing 82 is fluidically connected to the first downstream opening 62. In particular, the first downstream tubing 82 fluidically connects the first downstream opening 62 to the outside of the measurement cell 12 to evacuate first F1 and/or second F2 fluid from the rock sample 28.

The second downstream tubing 84 is fluidically connected to the second downstream opening 64. In particular, the second downstream tubing 84 fluidically connects the second downstream opening to the outside of the measurement cell 12 to evacuate first fluid F1 from the rock sample 28 through the porous barrier element 18.

One of the upstream 74 and downstream 76 cap comprises a confinement tubing 85, configured for guiding confinement fluid CF into the confinement housing 27. In particular, the confinement tubing 85 opens on the confinement space 77. The confinement tubing 85 connects the fluid regulation arrangement 16 to the confinement space 77.

In the example of figure 2, the downstream cap 76 comprises the confinement tubing 85.

As seen above, the fluid regulation arrangement 16 comprises the pressure measurement device 44. The fluid regulation arrangement 16 also preferably comprises a feeding device 86, a first valve 88 and a second valve 90. The fluid regulation arrangement 16 also comprises a confinement pressuring device 91.

As seen above, the pressure measurement device 44 is fluidically connected with the rock sample 28 and in particular with the inlet face 30 of the rock sample 28.

The pressure measurement device 44 is configured to measure the pressure of the first fluid F1 and/or of the second fluid F2 in the rock sample 28, in particular at the inlet face 30 of the rock sample 28.

The feeding device 86 is configured for feeding the second fluid F2 in the first upstream opening 40, in particular through the first upstream tubing 78.

The feeding device 86 is thus configured for, through the feeding of the second fluid F2 in the first upstream opening 40, to flood the rock sample 28 with the second fluid F2.

As it will be presented later, the feeding device 86 is configured for either feeding the second fluid F2 with a constant flow, or for feeding the second fluid with a constant pressure.

When the feeding device 86 is configured for feeding the second fluid with a constant pressure, the feeding device is for example logically connected with the pressure measurement device 44, the feeding device 86 feeding the second fluid at a flowrate so that the pressure measured by the measuring device is constant.

In the embodiment presented in figures 1 and 2, the feeding device is for example a pump. In a non-illustrated embodiment, the feeding device comprises a centrifuge that is configured for feeding the second fluid F2 in the first upstream opening 40.

The first valve 88 is fluidically connected to the first downstream opening 62. The first valve 88 is configured for selectively freeing or blocking the first downstream opening 62. As presented in figure 1, the measurement system 10 comprises for example a first connection tube 92 connected to the first downstream tubing 82, the first valve 88 being installed on the first connection tube 92 and selectively freeing or blocking the connection tube 92 to free or block the first downstream opening 62.

The second valve 90 is fluidically connected to the second downstream opening 64. The second valve 90 is configured for selectively freeing or blocking the second downstream opening 64. As presented in figure 1, the measurement system 10 comprises for example a second connection tube 94 connected to the second downstream tubing 84, the second valve 90 being installed on the second connection tube 94 and selectively freeing or blocking the connection tube 94 to free or block the second downstream opening 64.

The confinement pressuring device 91 is fluidically connected to the confinement tubing 85. In particular, the confinement pressuring device 91 is configured for pressuring the confinement fluid CF in the confinement housing 27 through the confinement tubing 85.

As illustrated in figure 1, the inner structure fluid imaging device 14 is arranged at an outside of the rock properties measurement cell 12.

The inner structure fluid imaging device 14 is for example arranged around the confinement frame 72.

The inner structure fluid imaging device 14 is configured to evaluate fluids saturation in first fluid F1 and/or second fluid F2 along the rock sample axis A-A'.

The inner structure imaging device 14 is for example a microtomography imaging device or nuclear magnetic resonance imaging device.

A method 100 for preparing a rock sample with a target initial first fluid saturation profile S1iT along a rock sample axis A-A', implemented by the above-described measurement cell 12 and measurement system 10 will now be presented.

In some non-described embodiments, the above-described measurement cell 12 implements alternative methods for preparing a rock sample with a target initial first fluid saturation profile along the rock sample axis A-A'.

As above-mentioned, the first fluid F1 is for example water, such that the initial first fluid saturation S1i is an initial water saturation, also known as Swi, and the second fluid F2 is for example oil.

The method 100 comprises a providing step 110, followed by a primary flooding step 120 with the second fluid F2 and by a secondary flooding step 130 with the second fluid F2.

The method 100 further comprises, for example, prior to the providing step 110, a preliminary flooding step, where the rock sample 28 is completely saturated by the first fluid F1.

The initial first fluid saturation S1i in the rock sample 28 at the providing step 110 is then sensibly equal to 100%, inner cavities of the rock sample being saturated with the first fluid F1 only.

In the providing step 110, the rock sample 28 as above mentioned, saturated in the first fluid F1, is provided.

The primary flooding step 120 with the second fluid F2 comprises feeding the second fluid F2 in the rock sample 28 from the inlet face 30 and retrieving first fluid F1 and/or second fluid F2 from the first open channel 66.

During the primary flooding step 120, second fluid F2 is in particular fed in the rock properties measurement cell 12 by the feeding device 86.

The pressure in first fluid F1 and second fluid F2 is for example measured by the pressure measurement device 44 during the primary flooding step 120.

The flowrate of the second fluid F2 is for example controlled by the feeding device 86 during the primary flooding step. In a specific embodiment, the second fluid F2 is fed in the rock sample with a constant flowrate during the primary flooding. The flowrate of the second fluid F2 is for example controlled by the feeding device 86 to be constant during the primary flooding.

Alternatively, or additionally, the pressure of the second fluid F2 is controlled by the feeding device 86 during the primary flooding step 120. In a specific embodiment, the second fluid is fed in the rock sample with a constant pressure during the primary flooding step 120. The pressure of the second fluid F2 is for example controlled by the feeding device 86 in conjunction with the pressure measurement device 44 so that the pressure of the second fluid F2 is constant during the primary flooding 120.

The primary flooding 120 comprises maintaining the first channel 66 open. The primary flooding comprises for example to that end opening and/or maintaining in an open position the first valve 88 for freeing the first downstream opening 62.

The first fluid F1 and/or the second fluid F2 contained in the rock sample 28 is therefore pressed out of the rock sample by the feeding device 86 and flow freely from the outlet face 32 of the rock sample to be retrieved.

In a specific embodiment, the primary flooding 120 comprises blocking the second channel to close the second channel 68 and prevent any fluid to flow through the second channel 68. The primary flooding 120 comprises for example to that end closing and/or maintaining in a closed position the second valve 90 for blocking the second downstream opening 64.

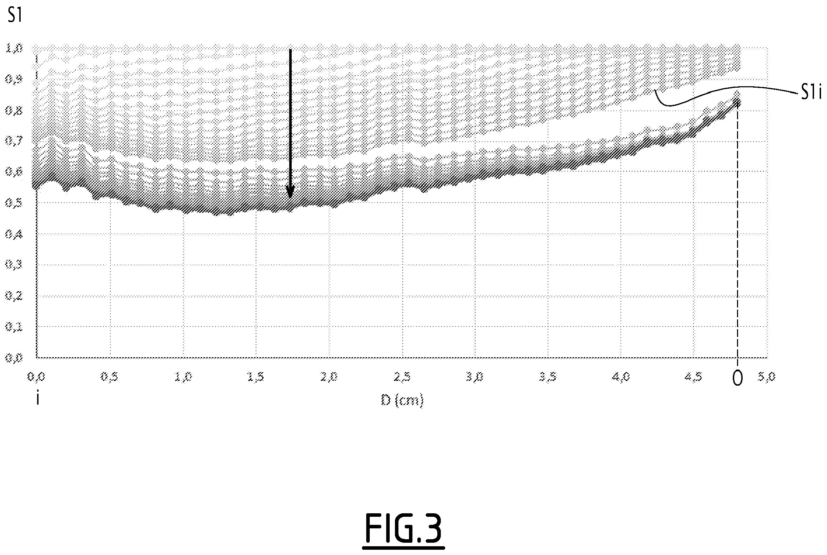

The evolution of the first fluid saturation profile S1i during the primary flooding 120 is illustrated in figure 3. The horizontal axis corresponds to the location along the rock sample axis, from position of the inlet face referenced i in figure 3, to the position of the outlet face, referenced o in figure 3. The vertical axis corresponds to the first fluid saturation S1, the first fluid saturation S1 along the rock sample axis forming at a defined time an initial first fluid saturation profile S1i. The arrow of figure 3 shows the general evolution of the initial first fluid saturation profile S1i during the primary flooding 120.

As visible from figure 3, the first fluid saturation profile S1i is decreasing over the primary flooding step 120 since first fluid F1 is being replaced in the rock sample by second fluid F2 being fed by the feeding device 86.

Once the primary flooding 120 is finished, the primary flooding 120 is stopped and the secondary flooding 130 of the rock sample with the second fluid F2 is started.

In some embodiments, the primary flooding is considered to be finished after a predetermined duration, the primary flooding 120 being stopped and the secondary flooding 130 started after the predetermined duration.

The predetermined duration is for example comprised between 6 hours and 92 hours.

In a preferred embodiment, the primary flooding 120 is stopped and the secondary flooding 130 is started when only second fluid F2 is retrieved from the first channel 66 during the primary flooding 120, that is when no first fluid F1 is being produced from the rock sample 28.

The secondary flooding 130 preferably immediately follows the first flooding 120.

The secondary flooding step 130 with the second fluid F2 comprises feeding the second fluid F2 in the rock sample 28 from the inlet face 30, and retrieving first fluid F1, from the second channel 68. In particular, since the second channel 68 is connected to the outlet face 32 of the rock sample 28 through the porous barrier element 18 that is permeable to the first fluid F1 and impervious to the second fluid F2, only first fluid F1 is retrieved from the rock sample during the secondary flooding 130.

The second fluid F2 is fed at a start of the secondary flooding 130 at a same pressure as the second fluid F2 is fed at an end of the primary flooding 120. The feeding device 86 is for example configured to feed the second fluid F2 at a same pressure at the end of the primary flooding 120 and at the start of the secondary flooding 130.

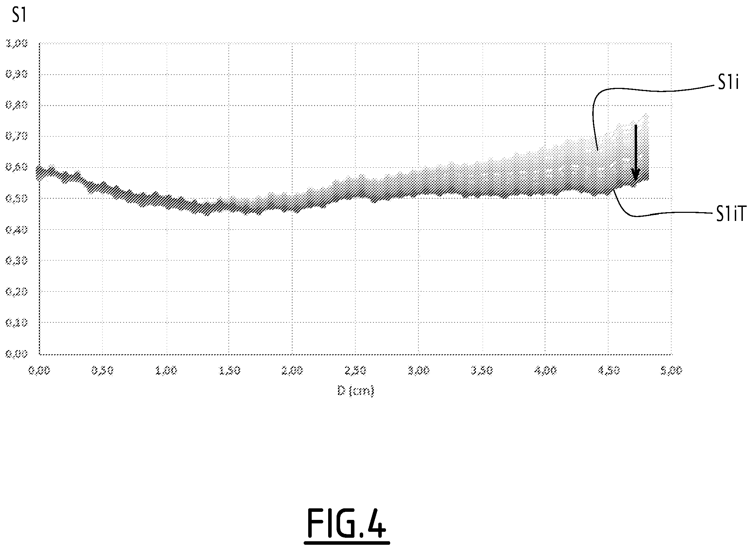

The secondary flooding 130 is configured to obtain the target initial first fluid saturation profile S1iT along the rock sample axis A-A', as shown in figure 4.

The secondary flooding 130 comprises blocking the first channel 66 to close the first channel 66 and prevent any fluid to flow through the first channel 66. The secondary flooding 130 comprises for example to that end closing and/or maintaining in a closed position the first valve 88 for blocking the first downstream opening 62. For example, the primary flooding 120 is stopped and the second flooding 130 is started by said blocking of the first channel 66 to close the first channel 66 and prevent any fluid to flow through the first channel 66.

When the primary flooding 120 comprises blocking the second channel 68, the secondary flooding 130 comprises opening the second channel 68. The secondary flooding 130 comprises for example to that end opening and/or maintaining in an opened position the second valve 90 for freeing the second downstream opening 64.

The second fluid F2 is fed in the rock sample 28 with a constant pressure during the secondary flooding step 130. The pressure of the second fluid F2 is preferably controlled by the feeding device 86 during the secondary flooding step 130.The pressure of the second fluid F2 is for example controlled by the feeding device 86 in conjunction with the pressure measurement device 44 so that the pressure of the second fluid F2 is constant during the secondary flooding 130.

The evolution of the first fluid saturation S1i during the secondary flooding 130 is illustrated in figure 4. The horizontal axis corresponds to the location along the rock sample axis, from position of the inlet face referenced i in figure 4, to the position of the outlet face, referenced in figure 4. The vertical axis corresponds to the first fluid saturation, the first fluid saturation S1 along the rock sample axis forming at a defined time an initial first fluid saturation profile S1i. The arrow of figure 4 shows the general evolution of the first fluid saturation profile S1i during the secondary flooding.

As visible from figure 4, the first fluid saturation profile S1i is further decreasing over the secondary flooding step 130 since first fluid F1 is being replaced in the rock sample by second fluid F2 being fed by the feeding device 86.

As visible from figure 4 the initial first fluid profile S1i along the rock sample axis is homogenized during the secondary flooding step so that the value of the first fluid saturation profile S1 along the rock sample axis A-A' is substantially constant.

Upon reaching the target initial first fluid saturation S1iT, the secondary flooding 130 is finished, the rock sample being finally prepared with the S1iT target initial first fluid saturation profile along the rock sample axis A-A'.

As presented on figure 4, the target initial first fluid saturation profile is for example above 40% along the rock sample axis A-A'.

The first fluid saturation profile S1i along the rock sample axis A-A' is for example monitored by nuclear magnetic resonance imaging during primary flooding and/or secondary flooding. Alternatively, or in complement, a first fluid F1 propagation within the rock sample 28 is for example monitored by microtomography during primary flooding 120 and/or secondary flooding 130.

The first fluid saturation profile S1i along the rock sample axis A-A' and/or the first fluid F1 propagation within the rock sample is in particular monitored with the inner structure imaging device 14.

Provision of a downstream diffuser 22 defining a downstream cavity and of a sealing gasket arranged around the downstream segment 50 of the porous element 18, between said downstream segment 50 and the downstream diffuser 22, is especially advantageous since this allows ensuring fluid-tightness without relying on an axial strain between the porous element 18 and the downstream diffuser 22, thus reducing the risk of damaging the porous element 18.

The confinement housing arranged 27 around the sleeve 24 allows pressuring the sleeve around the rock sample 28 with is especially advantageous to ensure a good pressuring of the fluids F1, F2 within the rock sample 28, ensuring properly establishing the first fluid saturation profile S1i along the rock sample axis A-A'.

The materials used for confinement housing 27 and for the sleeve 24 are especially advantageous since they are transparent to inner structure fluid imaging, allowing monitoring the establishment of the first fluid saturation profile S1i along the rock sample axis A-A'.

A downstream side 38 of the upstream diffuser 20 comprising at least a groove connected to the first upstream opening 40 is especially advantageous since this allows a more homogenous flooding of the rock sample 28.

A rock properties measurement sell 12 comprising a second upstream opening 42, configured for fluidically connecting the rock sample 28 with a pressure measurement device 44 allows measuring fluidic pressure within the rock sample 28 during the flooding, ensuring the pressure induced by the feeding device 86 allows a proper establishment of the first fluid saturation profile S1i.

The use of first 88 and second valve 90 as above described provides effective and simple means for switching between the primary flooding 120 and the secondary flooding 130.

The use of an inner structure fluid imaging device 14, in particular a microtomography imaging device or a nuclear magnetic resonance imaging device, is especially useful for monitoring the establishment of the first fluid saturation profile S1i along the rock sample axis A-A'.

## Claims

1. Rock properties measurement cell (12), configured for receiving a rock sample (28) to carry out inner structure imaging of at least a fluid contained in said rock sample (28), the measurement cell (12) comprising:
- a monolithic porous barrier element (18), permeable to a first fluid (F1) and impervious to a second fluid (F2), comprising:
+ an upstream segment (48) of a first diameter (D1), the upstream segment (48) defining an upstream face (52) configured to cooperate with the rock sample (28),
+ a downstream segment (50) of a second diameter (D2) smaller than the first diameter (D1), the upstream (48) and downstream (50) segments defining together a downstream face (54) opposed to the upstream face (52), and
+ a bypass (56) connecting the upstream face (52) and the downstream face (54) through the upstream (48) and the downstream (50) segments;
- a downstream diffuser (22), comprising:
+ a first downstream opening (62), delimiting, together with the bypass (56), a first channel (66) configured for retrieving the first (F1) and/or the second (F2) fluid from the rock sample (28),
+ a second downstream opening (64) forming a second channel (68), opening facing on the downstream face (54) of the upstream segment (48) of the porous barrier element (18) and being configured for retrieving the first fluid (F1) from the rock sample (28),
- an upstream diffuser (20) comprising a first upstream opening (40) configured for guiding the second fluid (F2) into the rock sample (28) ; and
- a sleeve (24) arranged around the upstream diffuser (20), the downstream diffuser (22) and the porous barrier element (18),
the upstream diffuser (20), the porous barrier element (18) and the sleeve (24) delimiting together a measurement chamber (34) configured for receiving the rock sample (28).

2. Rock properties measurement cell (12) according to claim 1, wherein the downstream diffuser (22) defines a cavity (61), the downstream segment (50) of the monolithic porous barrier element (18) being received in the cavity (61), the measurement cell (12) comprising a sealing gasket (26) arranged around the downstream segment (50) of the porous element (18), between said downstream segment (50) and the downstream diffuser (22).

3. Rock properties measurement cell (12) according to claim 1 or 2, comprising a confinement housing (27) arranged around the sleeve (24), the confinement housing (27) being configured for receiving a confinement fluid (CF) to apply pressure to the sleeve (24) around the upstream diffuser (20), the downstream diffuser (22), the porous barrier element (18) and the rock sample (28) received in the measurement chamber (34).

4. Rock properties measurement cell (12) according to claim 3, wherein the confinement housing (27) comprises:
- a upstream cap (74) comprising a first upstream tubing (78) fluidically connected to the first upstream opening (40),
- a downstream cap (76) comprising:
+ a first downstream tubing (82) fluidically connected to the first downstream opening (62),
+ a second downstream tubing (84) fluidically connected to the second downstream opening (64),
one of the upstream (74) and downstream (76) cap comprising a confinement tubing (85), configured for guiding the confinement fluid (CF) into the confinement housing (27).

5. Rock properties measurement cell (12) according to claim 3 or 4, wherein the confinement housing (27) is made of a poly ketone polymer, in particular polyether ether ketone.

6. Rock properties measurement cell (12) according to any of the preceding claims, wherein the sleeve (24) is made of fluorocarbon-based fluoroelastomer.

7. Rock properties measurement cell (12) according to any of the preceding claims, wherein an inner diameter of the sleeve (24) is comprised between 8mm and 75mm.

8. Rock properties measurement cell (12) according to any of the preceding claims, wherein a downstream side (38) of the upstream diffuser (20) comprises at least a groove connected to the first upstream opening (40), the groove being configured to guide the second fluid (F2) along an inlet face (30) of the rock sample (28).

9. Rock properties measurement cell (12) according to any of the preceding claims, wherein the upstream diffuser (20) comprises a second upstream opening (42), configured for fluidically connecting the rock sample (28) with a pressure measurement device (44).

10. Rock properties measurement system (10) comprising a rock properties measurement cell (12) according to any of the claims 1 to 9, wherein the system (10) comprises a feeding device (86), configured for feeding the second fluid (F2) in the first upstream opening (40).

11. Rock properties measurement system (10) according to claim 10, wherein the system (10) comprises:
- a first valve (88), fluidically connected to first downstream opening (62), and configured for selectively freeing or blocking the first downstream opening (62), and
- a second valve (90), fluidically connected to second downstream opening (64), and configured for selectively freeing or blocking the second downstream opening (64).

12. Rock properties measurement system (10) according to claims 10 or 11, wherein the system (10) comprises an inner structure fluid imaging device (14) arranged at an outside of the rock properties measurement cell (12).

13. Rock properties measurement system (10) according to claim 12, wherein the inner structure imaging device (14) is a microtomography imaging device or nuclear magnetic resonance imaging device.

## Patentansprüche

1. Messzelle (12) zur Messung von Gesteinseigenschaften, eingerichtet zum Aufnehmen einer Gesteinsprobe (28), um eine Abbildung der inneren Struktur mindestens eines in der Gesteinsprobe (28) enthaltenen Fluids durchzuführen, wobei die Messzelle (12) umfasst:
- ein monolithisches poröses Sperrelement (18), durchlässig für ein erstes Fluid (F1) und undurchlässig für ein zweites Fluid (F2), umfassend:
+ ein stromaufwärtiges Segment (48) mit einem ersten Durchmesser (D1), wobei das stromaufwärtige Segment (48) eine stromaufwärtige Fläche (52) definiert, die eingerichtet ist, mit der Gesteinsprobe (28) zusammenzuwirken,
+ ein stromabwärtiges Segment (50) mit einem zweiten Durchmesser (D2), der kleiner als der erste Durchmesser (D1) ist, wobei das stromaufwärtige (48) und das stromabwärtige (50) Segment zusammen eine der stromaufwärtigen Fläche (52) gegenüberliegende stromabwärtige Fläche (54) definieren, und
+ einen Bypass (56), der die stromaufwärtige Fläche (52) und die stromabwärtige Fläche (54) durch das stromaufwärtige (48) und das stromabwärtige (50) Segment verbindet;
- einen stromabwärtigen Diffusor (22), umfassend:
+ eine erste stromabwärtige Öffnung (62), die zusammen mit dem Bypass (56) einen ersten Kanal (66) begrenzt, der eingerichtet ist, das erste (F1) und/oder das zweite (F2) Fluid aus der Gesteinsprobe (28) zurückzugewinnen,
+ eine zweite stromabwärtige Öffnung (64), die einen zweiten Kanal (68) bildet, der der stromabwärtigen Fläche (54) des stromaufwärtigen Segments (48) des porösen Sperrelements (18) zugewandt mündet und eingerichtet ist, das erste Fluid (F1) aus der Gesteinsprobe (28) zurückzugewinnen,
- einen stromaufwärtigen Diffusor (20), umfassend eine erste stromaufwärtige Öffnung (40), die eingerichtet ist, das zweite Fluid (F2) in die Gesteinsprobe (28) zu führen ; und
- eine Hülse (24), die um den stromaufwärtigen Diffusor (20), den stromabwärtigen Diffusor (22) und das poröse Sperrelement (18) angeordnet ist,
wobei der stromaufwärtige Diffusor (20), das poröse Sperrelement (18) und die Hülse (24) zusammen eine Messkammer (34) begrenzen, die zum Aufnehmen der Gesteinsprobe (28) eingerichtet ist.

2. Messzelle (12) zur Messung von Gesteinseigenschaften nach Anspruch 1, wobei der stromabwärtige Diffusor (22) einen Hohlraum (61) definiert, wobei das stromabwärtige Segment (50) des monolithischen porösen Sperrelements (18) in dem Hohlraum (61) aufgenommen ist, wobei die Messzelle (12) eine Dichtung (26) umfasst, die um das stromabwärtige Segment (50) des porösen Elements (18) zwischen dem stromabwärtigen Segment (50) und dem stromabwärtigen Diffusor (22) angeordnet ist.

3. Messzelle (12) zur Messung von Gesteinseigenschaften nach Anspruch 1 oder 2, umfassend ein um die Hülse (24) angeordnetes Einschlussgehäuse (27), wobei das Einschlussgehäuse (27) eingerichtet ist, ein Einschlussfluid (CF) aufzunehmen, um Druck auf die Hülse (24) um den stromaufwärtigen Diffusor (20), den stromabwärtigen Diffusor (22), das poröse Sperrelement (18) und die in der Messkammer (34) aufgenommene Gesteinsprobe (28) auszuüben.

4. Messzelle (12) zur Messung von Gesteinseigenschaften nach Anspruch 3, wobei das Einschlussgehäuse (27) umfasst:
- eine stromaufwärtige Kappe (74), umfassend eine erste stromaufwärtige Leitung (78), die fluidisch mit der ersten stromaufwärtigen Öffnung (40) verbunden ist,
- eine stromabwärtige Kappe (76), umfassend:
+ eine erste stromabwärtige Leitung (82), die fluidisch mit der ersten stromabwärtigen Öffnung (62) verbunden ist,
+ eine zweite stromabwärtige Leitung (84), die fluidisch mit der zweiten stromabwärtigen Öffnung (64) verbunden ist,
wobei eine der stromaufwärtigen (74) und stromabwärtigen (76) Kappen eine Einschlussleitung (85) umfasst, die eingerichtet ist, das Einschlussfluid (CF) in das Einschlussgehäuse (27) zu führen.

5. Messzelle (12) zur Messung von Gesteinseigenschaften nach Anspruch 3 oder 4, wobei das Einschlussgehäuse (27) aus einem Polyketonpolymer, insbesondere Polyetheretherketon, hergestellt ist.

6. Messzelle (12) zur Messung von Gesteinseigenschaften nach einem der vorhergehenden Ansprüche, wobei die Hülse (24) aus einem Fluorelastomer auf Fluorkohlenstoffbasis hergestellt ist.

7. Messzelle (12) zur Messung von Gesteinseigenschaften nach einem der vorhergehenden Ansprüche, wobei ein Innendurchmesser der Hülse (24) zwischen 8mm und 75mm liegt.

8. Messzelle (12) zur Messung von Gesteinseigenschaften nach einem der vorhergehenden Ansprüche, wobei eine stromabwärtige Seite (38) des stromaufwärtigen Diffusors (20) mindestens eine mit der ersten stromaufwärtigen Öffnung (40) verbundene Nut umfasst, wobei die Nut eingerichtet ist, das zweite Fluid (F2) entlang einer Einlassfläche (30) der Gesteinsprobe (28) zu führen.

9. Messzelle (12) zur Messung von Gesteinseigenschaften nach einem der vorhergehenden Ansprüche, wobei der stromaufwärtige Diffusor (20) eine zweite stromaufwärtige Öffnung (42) umfasst, die eingerichtet ist, die Gesteinsprobe (28) fluidisch mit einer Druckmessvorrichtung (44) zu verbinden.

10. Messsystem (10) zur Messung von Gesteinseigenschaften, umfassend eine Messzelle (12) zur Messung von Gesteinseigenschaften nach einem der Ansprüche 1 bis 9, wobei das System (10) eine Zuführvorrichtung (86) umfasst, die eingerichtet ist, das zweite Fluid (F2) in die erste stromaufwärtige Öffnung (40) zuzuführen.

11. Messsystem (10) zur Messung von Gesteinseigenschaften nach Anspruch 10, wobei das System (10) umfasst:
- ein erstes Ventil (88), das fluidisch mit der ersten stromabwärtigen Öffnung (62) verbunden und eingerichtet ist, die erste stromabwärtige Öffnung (62) selektiv freizugeben oder zu blockieren, und
- ein zweites Ventil (90), das fluidisch mit der zweiten stromabwärtigen Öffnung (64) verbunden und eingerichtet ist, die zweite stromabwärtige Öffnung (64) selektiv freizugeben oder zu blockieren.

12. Messsystem (10) zur Messung von Gesteinseigenschaften nach Anspruch 10 oder 11, wobei das System (10) eine Vorrichtung (14) zur Fluidabbildung der inneren Struktur umfasst, die an einer Außenseite der Messzelle (12) zur Messung von Gesteinseigenschaften angeordnet ist.

13. Messsystem (10) zur Messung von Gesteinseigenschaften nach Anspruch 12, wobei die Vorrichtung (14) zur Abbildung der inneren Struktur eine Mikro-tomographie-Abbildungsvorrichtung oder eine Magnetresonanz-Abbildungsvorrichtung ist.

## Revendications

1. Cellule de mesure des propriétés de la roche (12), configurée pour recevoir un échantillon de roche (28) afin de réaliser une imagerie de structure interne d'au moins un fluide contenu dans ledit échantillon de roche (28), la cellule de mesure (12) comprenant :
- un élément barrière poreux monolithique (18), perméable à un premier fluide (F1) et imperméable à un second fluide (F2), comprenant :
+ un segment amont (48) d'un premier diamètre (D1), le segment amont (48) définissant une face amont (52) configurée pour coopérer avec l'échantillon de roche (28),
+ un segment aval (50) d'un second diamètre (D2) inférieur au premier diamètre (D1), les segments amont (48) et aval (50) définissant ensemble une face aval (54) opposée à la face amont (52), et
+ un bypass (56) reliant la face amont (52) et la face aval (54) à travers les segments amont (48) et aval (50) ;
- un diffuseur aval (22), comprenant :
+ une première ouverture aval (62), délimitant, conjointement avec le bypass (56), un premier canal (66) configuré pour récupérer le premier fluide (F1) et/ou le second fluide (F2) depuis l'échantillon de roche (28),
+ une seconde ouverture aval (64) formant un second canal (68), s'ouvrant en regard de la face aval (54) du segment amont (48) de l'élément barrière poreux (18) et étant configurée pour récupérer le premier fluide (F1) depuis l'échantillon de roche (28),
- un diffuseur amont (20) comprenant une première ouverture amont (40) configurée pour guider le second fluide (F2) dans l'échantillon de roche (28) ; et
- un manchon (24) agencé autour du diffuseur amont (20), du diffuseur aval (22) et de l'élément barrière poreux (18),
le diffuseur amont (20), l'élément barrière poreux (18) et le manchon (24) délimitant ensemble une chambre de mesure (34) configurée pour recevoir l'échantillon de roche (28).

2. Cellule de mesure des propriétés de la roche (12) selon la revendication 1, dans laquelle le diffuseur aval (22) définit une cavité (61), le segment aval (50) de l'élément barrière poreux monolithique (18) étant reçu dans la cavité (61), la cellule de mesure (12) comprenant un joint d'étanchéité (26) agencé autour du segment aval (50) de l'élément poreux (18), entre ledit segment aval (50) et le diffuseur aval (22).

3. Cellule de mesure des propriétés de la roche (12) selon la revendication 1 ou 2, comprenant un boîtier de confinement (27) agencé autour du manchon (24), le boîtier de confinement (27) étant configuré pour recevoir un fluide de confinement (CF) afin d'appliquer une pression au manchon (24) autour du diffuseur amont (20), du diffuseur aval (22), de l'élément barrière poreux (18) et de l'échantillon de roche (28) reçu dans la chambre de mesure (34).

4. Cellule de mesure des propriétés de la roche (12) selon la revendication 3, dans laquelle le boîtier de confinement (27) comprend :
- un capuchon amont (74) comprenant un premier tube amont (78) raccordé fluidiquement à la première ouverture amont (40),
- un capuchon aval (76) comprenant :
+ un premier tube aval (82) raccordé fluidiquement à la première ouverture aval (62),
+ un second tube aval (84) raccordé fluidiquement à la seconde ouverture aval (64),
l'un des capuchons amont (74) et aval (76) comprenant un tube de confinement (85), configuré pour guider le fluide de confinement (CF) dans le boîtier de confinement (27).

5. Cellule de mesure des propriétés de la roche (12) selon la revendication 3 ou 4, dans laquelle le boîtier de confinement (27) est constitué d'un polymère polycétone, en particulier de polyétheréthercétone.

6. Cellule de mesure des propriétés de la roche (12) selon l'une quelconque des revendications précédentes, dans laquelle le manchon (24) est constitué d'un fluoroélastomère à base de fluorocarbone.

7. Cellule de mesure des propriétés de la roche (12) selon l'une quelconque des revendications précédentes, dans laquelle un diamètre intérieur du manchon (24) est compris entre 8mm et 75mm.

8. Cellule de mesure des propriétés de la roche (12) selon l'une quelconque des revendications précédentes, dans laquelle un côté aval (38) du diffuseur amont (20) comprend au moins une rainure reliée à la première ouverture amont (40), la rainure étant configurée pour guider le second fluide (F2) le long d'une face d'entrée (30) de l'échantillon de roche (28).

9. Cellule de mesure des propriétés de la roche (12) selon l'une quelconque des revendications précédentes, dans laquelle le diffuseur amont (20) comprend une seconde ouverture amont (42), configurée pour relier fluidiquement l'échantillon de roche (28) à un dispositif de mesure de pression (44).

10. Système de mesure des propriétés de la roche (10) comprenant une cellule de mesure des propriétés de la roche (12) selon l'une quelconque des revendications 1 à 9, dans lequel le système (10) comprend un dispositif d'alimentation (86), configuré pour alimenter la seconde fluide (F2) dans la première ouverture amont (40).

11. Système de mesure des propriétés de la roche (10) selon la revendication 10, dans lequel le système (10) comprend :
- une première vanne (88), raccordée fluidiquement à la première ouverture aval (62), et configurée pour libérer ou bloquer sélectivement la première ouverture aval (62), et
- une seconde vanne (90), raccordée fluidiquement à la seconde ouverture aval (64), et configurée pour libérer ou bloquer sélectivement la seconde ouverture aval (64).

12. Système de mesure des propriétés de la roche (10) selon la revendication 10 ou 11, dans lequel le système (10) comprend un dispositif d'imagerie de fluide de structure interne (14) agencé à l'extérieur de la cellule de mesure des propriétés de la roche (12).

13. Système de mesure des propriétés de la roche (10) selon la revendication 12, dans lequel le dispositif d'imagerie de structure interne (14) est un dispositif d'imagerie par microtomographie ou un dispositif d'imagerie par résonance magnétique nucléaire.
